# FASCICULE DE BREVET EUROPEEN

(11) **EP 1 053 727 B1**
(45) Date de publication et mention de la délivrance du brevet: **08.10.2003**
(21) Numéro de dépôt: 00440150.1
(22) Date de dépôt: 19.05.2000
(51) Int. Cl.: A61F 11/00, A61F 13/38

(54) **Instrument manuel de curetage et son procédé de fabrication**
Handreinigungsinstrument und Verfahren zu dessen Herstellung
Manual scraping tool and process for its manufacturing

(30) Priorité: 20.05.1999 FR 9906547
(43) Date de publication de la demande: 22.11.2000
(73) Titulaire: Sittler, Jean-Pierre, 67230 Herbsheim (FR)
(72) Inventeur: Sittler, Jean-Pierre, 67230 Herbsheim (FR)
(74) Mandataire: Metz, Paul

(56) Documents cités:
- DE-A- 19 610 998
- DE-C- 557 917
- FR-A- 2 249 640
- FR-A- 2 760 633
- GB-A- 334 132
- US-A- 1 425 776
- US-A- 3 967 338
- US-A- 4 329 990

## Description

La présente invention concerne un instrument manuel de curetage, pour le curetage et le nettoyage de conduits ou de cavités anatomiques. Cet instrument est particulièrement adapté pour le nettoyage du conduit auditif.

L'invention se rapporte également au procédé de fabrication de cet instrument de curetage.

Pour des questions d'hygiène, il est nécessaire d'évacuer la cire ou le cérumen contenu dans le conduit auditif des oreilles. Pour cela, on utilise habituellement un instrument de nettoyage appelé communément « coton-tige ». Un coton-tige est un dispositif constitué d'un bâtonnet semi-rigide comportant à ses deux extrémités un tampon de coton.

Au cours du nettoyage, le coton-tige est introduit dans le conduit auditif. On lui applique alors un mouvement de rotation sur lui-même, qui permet au tampon de coton d'essuyer les parois du conduit.

Cependant, les cotons-tiges possèdent un inconvénient. En effet, le tampon de coton placé à l'extrémité du bâtonnet présente une section axiale sensiblement ovoïde. Le bout rond et relativement gros du tampon de coton tend à repousser une partie du cérumen au fond du conduit auditif. Le cérumen ainsi repoussé s'accumule, formant progressivement un bouchon pouvant causer une surdité partielle.

Pour remédier à cet inconvénient, la publication de brevet français n° 2.760.633 enseigne un instrument manuel de curetage, du type cure-oreille, permettant de détacher et d'extraire la cire ou le cérumen incrusté sur les parois du conduit auditif. L'instrument est formé d'un bâtonnet de manoeuvre présentant à l'une au moins de ses extrémités un moyen de curetage en forme de bulbe. Le moyen de curetage se compose d'une pluralité de lamelles reliées entre elles au niveau de l'une de leurs extrémités, leur autre extrémité étant solidarisée à l'extrémité du bâtonnet. Ces lamelles présentent des chants dont les bords d'attaque sont conformés en arêtes racleuses.

Les lamelles sont réalisées en un matériau plastique ou analogue relativement souple. Ainsi, lorsque l'utilisateur introduit l'instrument dans son conduit auditif, les lamelles se déforment élastiquement pour s'adapter parfaitement aux formes et dimensions du conduit. Cet effet s'amplifie lorsque l'extrémité de l'instrument, correspondant au point de regroupement des lamelles, arrive en butée contre un obstacle ou une paroi transversale au conduit. L'utilisateur imprime alors au bâtonnet un mouvement de rotation sur lui-même, qui permet aux arêtes des lamelles de venir racler les parois du conduit et d'en détacher les dépôts de cérumen. Lorsque l'utilisateur retire l'instrument de curetage tout en poursuivant son mouvement de rotation, les dépôts de cérumen préalablement détachés sont entraînés vers la sortie par l'effet du mouvement hélicoïdal provenant de la conformation du bulbe en lamelles aptes à se vriller ou à se torsader.

Cet instrument racleur de curetage présente des avantages très intéressants par rapport à un coton-tige classique. Cependant son procédé de fabrication n'a pas été décrit dans cette demande.

Pour connaître un succès commercial, un instrument manuel de curetage pour conduit auditif doit avoir un prix de vente unitaire très faible. En effet, son utilisation est unique et de courte durée. De plus, il est en concurrence sur le marché avec les cotons-tiges classiques dont le prix de vente unitaire est extrêmement modique. Chaque composante du prix de revient, se répercutant directement sur le prix de vente, doit donc être étudiée pour la réduire au minimum. Le choix de la matière première et du procédé de fabrication doit tenir compte en priorité de cette contrainte. Le procédé de fabrication doit être simple pour pouvoir être facilement mécanisé et automatisé. Le coût des machines et le nombre d'opérateurs nécessaires doivent également être faibles. Les étapes du procédé doivent être rapides et leur nombre réduit afin de diminuer le temps de fabrication unitaire et par conséquent le prix de revient unitaire.

L'instrument de curetage décrit dans la demande française FR 2.760.633, possède une structure peu adaptée à une fabrication industrielle. En effet, le moyen de curetage comporte des parties creuses très peu accessibles de l'extérieur qui rendent un procédé de moulage très compliqué, voire impossible.

La demande européenne EP 0.875.221 propose un procédé de fabrication pour cet instrument de curetage. Au cours d'une première étape, on réalise par moulage-injection le bâtonnet prolongé à son ou ses extrémités par les lamelles racleuses. Des prolongations sous forme de brins sont prévues à l'autre extrémité des lamelles. Au cours d'une seconde étape, ces brins sont rassemblés afin de réunir l'extrémité des lamelles. Les lamelles sont ensuite mises en forme et solidarisées par soudage. Les brins sont alors coupés et détachés.

Ce procédé comporte plusieurs étapes et nécessite un outillage important et coûteux en plus du moule d'injection. Après le moulage, il faut rassembler les lamelles, leurs donner la forme d'un bulbe, les souder à l'aide d'un outil chauffant, puis couper les brins inutiles. L'automatisation d'un tel procédé n'est pas aisée et se traduit par un coût d'outillage, de personnel et d'entretien relativement important. De plus du fait de cette succession d'étapes, le temps de fabrication unitaire est relativement long, ce qui augmente encore le coût de fabrication.

Le but de l'invention est de fournir un instrument manuel de curetage amélioré, présentant tous les avantages de l'instrument décrit dans la demande française FR 2.760.633, mais possédant une structure beaucoup mieux adaptée à un procédé industriel de fabrication.

En effet, lorsqu'il est en position d'utilisation, l'instrument amélioré selon l'invention comporte comme l'instrument précédent un moyen de curetage conformé en bulbe à lamelles racleuses souples, permettant de détacher le cérumen du conduit auditif et de l'évacuer par entraînement hélicoïdal grâce à un effet de vrille ou torsade de ses lamelles.

Cependant la structure de l'instrument de curetage selon l'invention est telle, qu'il est beaucoup plus facile à fabriquer. L'instrument ne comporte aucune surface creuse ou forme difficile à mouler. Il est réalisé à partir d'un seul matériau et d'une seule pièce. Il peut, sans aucune difficulté, être produit en une seule étape par un procédé de moulage par injection. Ce procédé, connu de l'homme du métier, peut facilement être automatisé. Le procédé ne comportant qu'une seule étape, la vitesse unitaire de fabrication est grandement augmentée par rapport au procédé décrit précédemment. On peut ainsi très fortement diminuer le prix de revient unitaire d'un tel instrument de curetage et le rendre compétitif par rapport à un coton-tige classique.

Un autre but de l'invention est de fournir un instrument de curetage amélioré possédant à la fois les avantages du coton-tige et de l'instrument à lamelles racleuses de la demande FR 2.760.633.

Pour atteindre ces objectifs, l'instrument manuel de curetage selon la présente invention comporte les moyens suivants.

L'instrument est constitué d'un seul tenant par un bâtonnet tubulaire dont au moins une des extrémités est prolongée successivement par deux demi-coques, par exemple identiques, articulées entre elles par charnière, puis par un segment d'axe. En pliant la charnière, les deux demi-coques peuvent être rapportées l'une sur l'autre, constituant ainsi une coque complète en forme de bulbe. L'instrument de curetage est maintenu dans cette position par encliquetage ou engagement à force du segment d'axe dans une découpe longitudinale du bâtonnet. Chaque demi-coque est constituée de plusieurs lamelles longitudinales, réunies entre elles à leurs extrémités. Ces lamelles présentent des chants dont les bords d'attaque sont conformés en arêtes racleuses.

Après l'étape de moulage, on obtient un instrument présentant deux demi-coques ouvertes, pouvant éventuellement être vendu tel quel. Pour placer l'instrument en position d'utilisation, il suffit de plier la charnière et d'encliqueter le segment d'axe. Cette opération très simple peut être réalisée par le fabricant initial, par un intervenant intermédiaire ou même par l'utilisateur.

Lorsque l'instrument de curetage est placé en position d'utilisation, il possède une structure, en bulbe à lamelles racleuses, semblable à celle de l'instrument décrit dans la demande de brevet FR 2.760.633. Il présente donc les mêmes avantages de fonctionnement que cet instrument.

L'instrument de curetage amélioré présente de surcroît un avantage supplémentaire. En effet, l'espace intérieur du bulbe de curetage est libre et accessible lors de la fabrication, tant que les deux demi-coques n'ont pas été refermées. Il est de ce fait possible d'y placer un produit. On peut ainsi, par exemple, rajouter à l'intérieur du moyen de curetage une capsule libérant un produit absorbant sec ou imprégné, par exemple un tampon de coton hydrophile ou d'ouate de cellulose. Ce tampon permet de compléter le nettoyage du conduit en ajoutant au curetage réalisé par les lamelles, la libération d'un liquide et un essuyage similaire à celui des cotons-tiges et une absorption des liquides, écoulements, ou autres. L'instrument de curetage amélioré selon l'invention cumule ainsi les avantages du coton-tige et de l'instrument de curetage décrit précédemment.

Ce tampon peut également être imprégné d'une solution active, au stade de la fabrication de l'instrument de curetage ou juste avant son utilisation, par exemple par trempage par l'utilisateur -de la coque renfermant le tampon dans la solution active. On peut envisager l'utilisation de solutions très variées, pouvant, par exemple, avoir une activité nettoyante, de rinçage, désinfectante, traitante contre une affection quelconque.

Lorsque l'on introduit le moyen de curetage dans le conduit à nettoyer, les lamelles souples s'aplatissent pour s'adapter aux dimensions du conduit. De même l'extrémité de la coque s'écrase en cas de poussée sur un obstacle fixe ou une paroi transversale. L'espace intérieur du bulbe diminue alors et les lamelles exercent une pression sur le tampon imprégné. La solution active est ainsi libérée dans le conduit qu'elle tapisse d'un revêtement sensiblement uniforme au cours des opérations de rotation et de translation de l'instrument de curetage.

On peut imaginer sur ce principe de très nombreuses variantes, par exemple un instrument comportant un côté racleur et un côté imprégné, ou bien deux côtés imprégnés de deux solutions différentes, ou alors un côté renfermant à la place d'un tampon absorbant de petites capsules s'écrasant lors de l'utilisation de l'instrument afin de libérer la solution active qu'elles contiennent.

Il va de soi que l'invention n'est pas limitée dans ses applications à l'hygiène du conduit auditif. Un tel instrument de curetage est, par exemple, parfaitement adapté au curetage et au nettoyage d'autres conduits ou cavités anatomiques. Il peut également permettre d'appliquer, dans ces conduits ou cavités difficiles d'accès, des substances ou produits actifs, par exemple dans le cadre d'un traitement médical.

En plus de ses applications dans le domaine médical, paramédical ou le domaine de l'hygiène, l'instrument selon la présente invention peut être utilisé dans de nombreux autres domaines techniques, tels que par exemple le domaine du nettoyage. Il peut, par exemple, être utilisé pour nettoyer certaines pièces mécaniques ou certains endroits exiguës où il est difficile de faire pénétrer un instrument de nettoyage classique, tels que des rainures ou analogues.

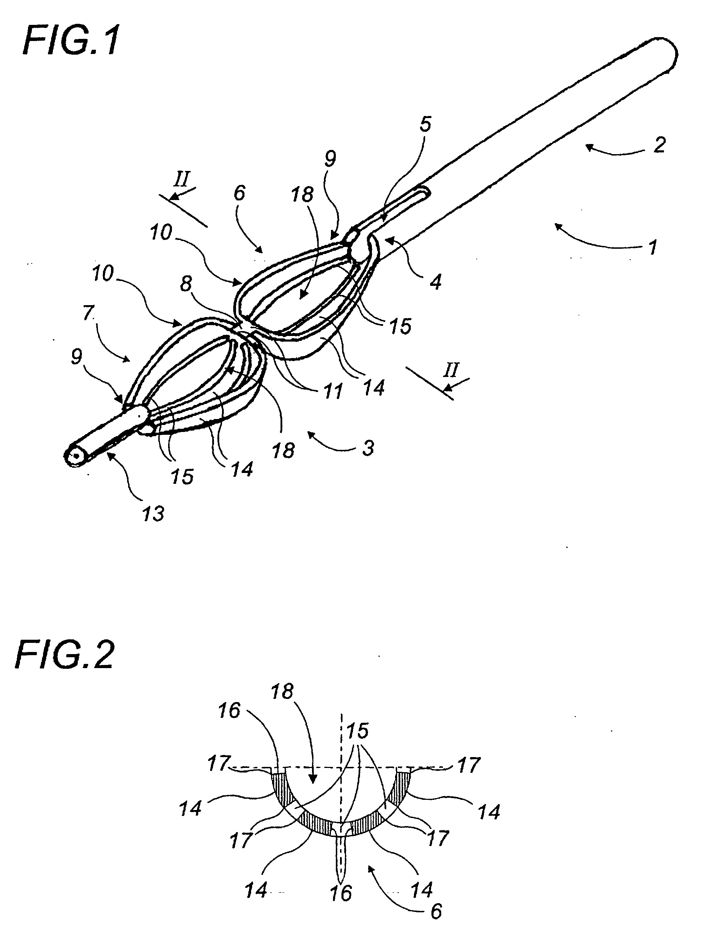

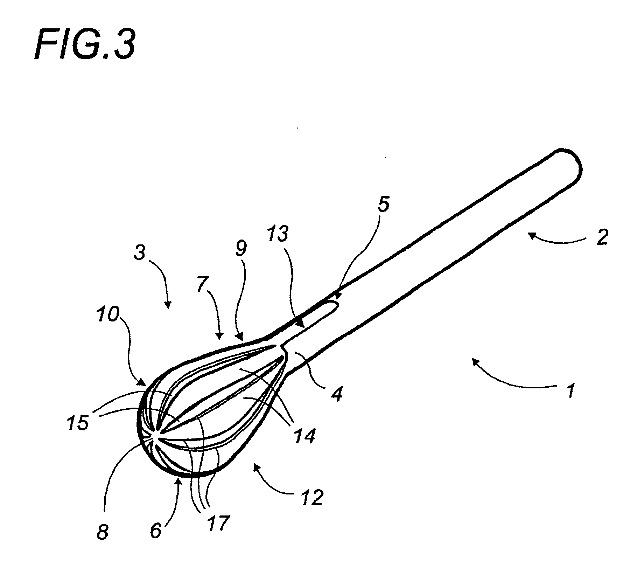

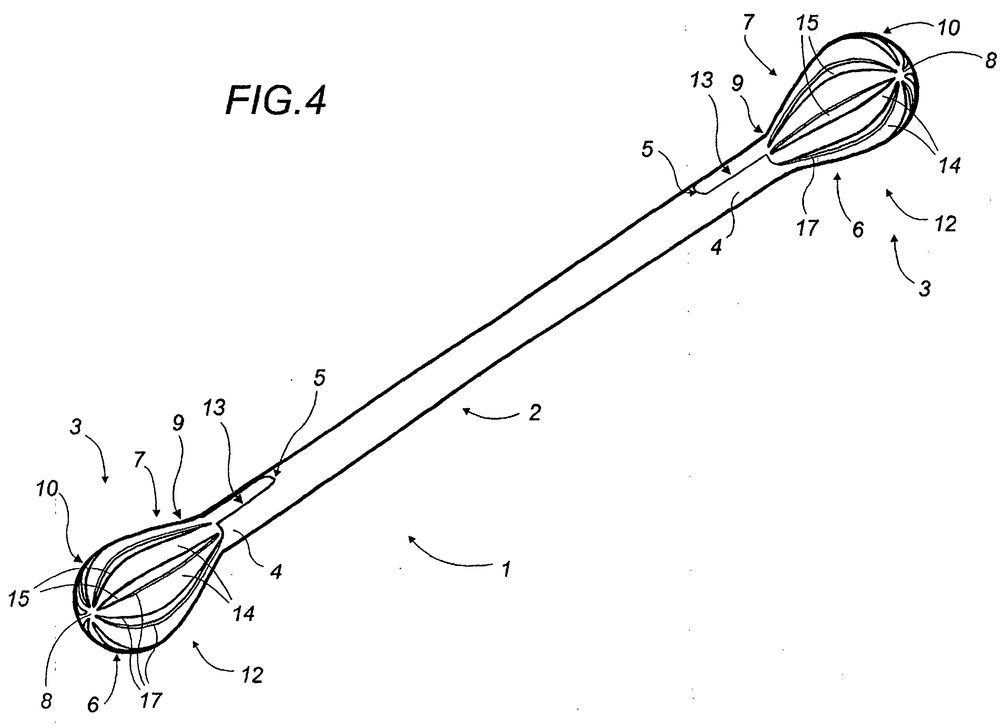

D'autres caractéristiques et avantages de l'invention apparaîtront à la lecture de la description détaillée qui va suivre, description faite en référence aux dessins annexés, dans lesquels :
. la figure 1 est une vue en perspective de l'instrument de curetage selon l'invention lorsque le moyen de curetage est ouvert et se présente sous la forme de deux demi-coques reliées entre elles par une charnière d'articulation ;
. la figure 2 est une vue en coupe transversale, selon la ligne II-II de la figure 1, d'une demi-coque du moyen de curetage, par exemple la demi-coque inférieure ;
. la figure 3 est une vue en perspective de l'instrument de curetage prêt à être utilisé, le moyen de curetage ayant été refermé et les deux demi-coques réunies formant un bulbe ;
. la figure 4 est une vue en perspective d'un autre mode de réalisation de l'instrument de curetage selon l'invention comprenant deux moyens de curetage refermés, situés aux deux extrémités d'un bâtonnet.

L'instrument de curetage selon la présente invention va maintenant être décrit de façon détaillée en référence aux figures 1 à 4. Les éléments équivalents, représentés sur les différentes figures, porteront les mêmes références numériques.

L'instrument de curetage selon l'invention, désigné dans *son* ensemble par la référence 1, est constitué d'un bâtonnet 2 de préhension et de manoeuvre et d'au moins un moyen de curetage 3 situé à l'une des extrémités 4 du bâtonnet 2. Le mode de réalisation représenté dans les figures 1 à 3 ne comprend qu'un seul moyen de curetage 3. De manière évidente, le bâtonnet peut comporter un moyen de curetage 3 à chacune de ses extrémités 4, sans sortir du cadre de la présente invention. Cet autre mode de réalisation, plus proche du coton tige classique, a été représenté à la figure 4.

L'instrument de curetage 1 est réalisé d'une seule pièce en matière plastique, par exemple par un procédé de moulage par injection.

Le bâtonnet 2 de manoeuvre est une tige de forme générale tubulaire permettant à l'utilisateur de tenir l'instrument de curetage et de lui imprimer les mouvements de translation et de rotation, adaptés à un nettoyage optimal, par exemple du conduit auditif.

Le bâtonnet 2 présente, à son ou ses extrémités 4 comportant un moyen de curetage 3, une découpe longitudinale 5 en fente selon l'axe du bâtonnet. Cette découpe 5 est débouchante et permet d'accéder à l'intérieur creux de la tige tubulaire constituant le bâtonnet 2.

Le moyen de curetage 3 comporte deux demi-coques 6 et 7 de mêmes dimensions, par exemple identiques, reliées entre elles par une articulation, par exemple à charnière 8, permettant la liaison articulée de l'une par rapport à l'autre. Les deux demi-coques 6 et 7 sont creuses et présentent une forme, par exemple de demi-bulbe, comprenant une base 9 étroite et une partie supérieure 10 large sensiblement arrondie.

L'instrument de curetage selon l'invention étant réalisé d'une seule pièce, la première demi-coque 6 constitue le prolongement de l'extrémité 4 du bâtonnet 2. La base 9 de cette demi-coque 6 se fond avec la demi-partie inférieure de l'extrémité 4 du bâtonnet 2. La partie supérieure 10 de la demi-coque 6 se prolonge dans sa partie centrale 11 par une zone 8, de forme sensiblement rectangulaire, se projetant vers l'extérieur et servant d'articulation de liaison, par exemple à charnière. La demi-coque 7 est reliée de façon symétrique par la zone centrale 11 de sa partie supérieure 10 au côté opposé de la zone de charnière 8. L'instrument de curetage selon la présente invention se compose donc d'une seule pièce et peut être fabriqué d'un seul tenant, par exemple par un procédé de moulage par injection.

La charnière 8 est constituée de façon classique d'une zone de matière plastique de plus faible épaisseur. Elle permet, par pliage, de rapporter la demi-coque 7 sur la demi-coque 6 et ainsi de constituer une coque 12 complète en forme générale en bulbe. L'instrument de curetage 1 selon la présente invention est alors en état d'utilisation.

La base 9 de la demi-coque extérieure 7 se prolonge par un segment d'axe 13 en bout de tige se projetant longitudinalement selon l'axe du bâtonnet 2. Le bout 13 d'axe est sensiblement cylindrique. Il présente un diamètre légèrement supérieur à la largeur de la découpe longitudinale 5 du bâtonnet 2 et une longueur inférieure ou égale à celle de cette même découpe 5.

Ce bout 13 d'axe permet de maintenir et de verrouiller l'instrument de curetage 1 en configuration d'utilisation. En effet, lorsque l'on plie la charnière 8 et que l'on rapporte la demi-coque 7 sur la demi-coque 6, le bout 13 d'axe se trouve au dessus de la découpe longitudinale 5 du bâtonnet 2. Le bout 13 d'axe, légèrement plus large que la découpe 5, peut être encliqueté à force dans la découpe 5 et ainsi introduit latéralement dans l'extrémité du bâtonnet 2. Le bout 13 d'axe ne peut alors plus que difficilement sortir du bâtonnet 2, verrouillant ainsi l'instrument de curetage en configuration d'utilisation. Cette configuration est illustrée à la figure 3 pour un instrument ne possédant qu'un moyen de curetage 3 et à la figure 4 pour un instrument en comportant deux.

Chacune des demi-coques 6 et 7 est constituée d'une pluralité de lamelles ou lanières longitudinales 14 plates, juxtaposées et séparées par des fentes longitudinales 15. Les lamelles 14 sont réunies entre elles à chacune de leurs extrémités à la base 9 et dans la zone centrale 11 de la partie supérieure 10 de chaque demi-coque 6 et 7.

La figure 2 représente la section transversale de l'une des demi-coques de l'instrument de curetage 1 selon l'invention. Il s'agit par exemple de la demi-coque inférieure 6. Dans le mode de réalisation représenté, la demi-coque 6 comporte quatre lamelles 14. Ce nombre peut évidemment varier dans les différentes réalisations de l'invention.

Chacune des lamelles 14 présente dans sa zone médiane une forme incurvée convexe et comporte des chants 16 dont les bords d'attaque sont conformés en arêtes vives 17 non blessantes. Ces arêtes 17 permettent de décoller et de racler le cérumen tapissant le conduit auditif à nettoyer.

Cette configuration en lamelles, réalisées en matière plastique ou analogue relativement élastique, apporte au moyen de curetage un caractère aisément déformable, lui permettant de s'adapter parfaitement aux dimensions du conduit à nettoyer et de s'aplatir en cas de contact de poussée avec un obstacle transversal fixe. Cependant, chacune des lamelles 14 présente une rigidité suffisante pour assurer un travail de raclage efficace.

L'invention se rapporte également au procédé de fabrication de l'instrument de curetage 1 décrit ci-dessus.

Le procédé de fabrication procède de l'idée générale inventive qui consiste à réaliser l'instrument de curetage 1 en une seule pièce d'un seul tenant au cours d'une seule étape de moulage par injection. On obtient ainsi l'instrument 1 tel qu'il est représenté à la figure 1. Cet instrument ne comporte pas de zone creuse fermée ou de forme difficile à réaliser. Il peut donc aisément être fabriqué industriellement, par un procédé automatisé de moulage par injection. Cette étape est rapide et économique. L'instrument de curetage 1 peut éventuellement être commercialisé sous cette forme.

On a, à ce moment de la fabrication, accès à l'espace intérieur 18 de la coque 12 en forme de bulbe constituant le moyen de curetage 3. Cet espace intérieur 18 peut être laissé vide, ou rempli avec un élément tel qu'un tampon en matière absorbante, par exemple un tampon de coton hydrophile ou d'ouate de cellulose ou autre produit. Ce tampon améliore l'efficacité de l'instrument de curetage car il permet de réaliser, en plus d'un raclage du cérumen par les lamelles, un essuyage et une absorption des écoulements. Le tampon peut éventuellement être imprégné d'une solution active.

La dernière étape consiste à refermer le moyen de curetage 3 en pliant la charnière 8 et à encliqueter le segment d'axe 13 dans la découpe 5 afin de verrouiller l'instrument 1 en position d'utilisation. L'instrument de curetage 1, prêt à l'emploi, est alors dans la configuration illustrée à la figure 3. Cette dernière étape ne présente aucune difficulté. Elle peut être réalisée dans une chaîne automatisée de fabrication, de manière simple et rapide, et sans ajouter de surcoût important par rapport au coût total de fabrication.

Il va de soi que l'invention n'est pas limitée dans ses applications au nettoyage des conduits auditifs et que diverses autres applications.

L'invention inclut notamment les instruments de curetage dont le moyen de curetage 3 est constitué de plus de deux portions de coque aptes à former une coque complète ou ceux dont les moyens de curetages présentent des ouvertures dont la forme est différente de celle des fentes longitudinales 15 représentées.

Il en est de même pour le procédé qui peut subir quelques variations sans sortir du cadre des revendications.

## Revendications

1. Instrument manuel de curetage formé d'un bâtonnet (2) et d'au moins un moyen de curetage (3) solidaire de l'une des extrémités (4) du bâtonnet (2) **caractérisé en ce que** le moyen de curetage (3) est constitué d'au moins deux parties de coque (6) et (7) présentant des ouvertures (15), pouvant être rapportées l'une sur l'autre de manière à former une coque creuse (12) complète et à délimiter un espace intérieur (18) libre, clos, susceptible d'être occupé par un élément absorbant, un matériau imprégné ou une réserve de produit, les parties de coque (6) et (7) étant maintenues dans cette position d'utilisation par un moyen de verrouillage (13, 5).

2. Instrument manuel de curetage selon la revendication 1 **caractérisé en ce que** les parties de coque (6) et (7) sont reliées entre elles par une liaison articulée.

3. Instrument manuel de curetage selon la revendication 2 **caractérisé en ce que** les parties de coque (6) et (7) sont reliées entre elles et articulées par charnière (8).

4. Instrument manuel de curetage selon la revendication 1 ou 2 **caractérisé en ce que** le moyen de verrouillage (13, 5) est un dispositif d'encliquetage.

5. Instrument manuel de curetage selon la revendication 4 **caractérisé en ce que** ce dispositif d'encliquetage est constitué par un bout (13) d'axe prolongeant l'une des portions de coque (7) devant être introduit à force dans une découpe (5) de l'extrémité (4) du bâtonnet (2) à laquelle est solidarisée une autre partie de coque (6).

6. Instrument manuel de curetage selon l'une quelconque des revendications précédentes **caractérisé en ce que** cet instrument de curetage est réalisé d'un seul tenant dans la même matière.

7. Instrument manuel de curetage selon l'une quelconque des revendications précédentes **caractérisé en ce que** le moyen de curetage (3), en configuration d'utilisation, est susceptible de déformation lorsqu'il est en contact frontal de butée.

8. Instrument manuel de curetage selon l'une quelconque des revendications précédentes **caractérisé en ce que** les ouvertures (15) sont des fentes longitudinales divisant les parties de coque (6) et (7) en lamelles (14) souples individualisées reliées entre elles à chacune de leurs extrémités.

9. Instrument manuel de curetage selon la revendication 8 **caractérisé en ce que** les lamelles (14) sont plates et présentent des chants (16) dont les bordures sont conformées en arêtes (17) par lesquelles elles viennent racler les parois du conduit à nettoyer.

10. Instrument manuel de curetage selon la revendication 8 ou 9 **caractérisé en ce que** les lamelles (14) présentent lors de la rotation du bâtonnet (2) sur lui-même un effet de torsade ou de vrille permettant une extraction par entraînement hélicoïdal des dépôts désincrustés.

11. Instrument manuel de curetage selon l'une quelconque des revendications précédentes **caractérisé en ce que** l'espace libre (18) intérieur aux parties de coque (6) et (7) renferme un élément absorbant.

12. Instrument manuel de curetage selon l'une quelconque des revendications précédentes **caractérisé en ce que** l'espace libre (18) intérieur aux parties de coque (6) et (7) renferme une substance active s'échappant de l'instrument de curetage lors de son l'utilisation.

13. Instrument manuel de curetage selon la revendication 12 **caractérisé en ce que** la substance active est imprégnée sur un matériau absorbant.

14. Instrument manuel de curetage selon la revendication 12 **caractérise en ce que** la substance active est renfermée dans un contenant et est libérée au cours de l'utilisation de l'instrument de curetage.

15. Procédé de fabrication d'un instrument manuel de curetage tel que revendiqué dans les revendications précédentes **caractérisé en ce que** l'on forme, d'un seul tenant et en une seule étape de moulage par injection, le bâtonnet (2) et le ou les moyen(s) de curetage (3).

16. Procédé selon la revendication 15 **caractérisé en ce que** l'on introduit un élément absorbant, un matériau imprégné ou une réserve de produit dans au moins un des espaces libres (18) intérieurs aux parties de coque (6) et (7).

17. Procédé selon la revendication 15 ou 16 **caractérisé en ce que** l'on referme le ou les espace(s) libre(s) intérieur(s) (18) en rapportant les parties de coque (6) et (7) les unes à côté des autres et que l'on bloque ces parties de coque (6) et (7) en configuration d'utilisation à l'aide du moyen de verrouillage (13, 5).

## Patentansprüche

1. Handreinigungsinstrument mit einem Stäbchen (2) und wenigstens einem mit einem der Enden (4) des Stäbchens (2) verbundenen Reinigungsteil (3), **dadurch gekennzeichnet, dass** das Reinigungsteil (3) aus wenigstens zwei Schalenteilen (6) und (7) besteht, die Öffnungen (15) aufweisen und so miteinander in Verbindung bringbar sind, dass ein vollständig umschlossener Hohlkörper (12) ausgebildet und ein freier, abgetrennter, durchlässiger Innenraum (18) abgegrenzt ist, der mit einem absorbierenden Element, einem getränkten Material oder einem Produktvorrat bestückbar ist, wobei die Schalenteile (6) und (7) durch ein Verriegelungsteil (13, 5) in dieser Anwendungsstellung gehalten sind.

2. Handreinigungsinstrument nach Anspruch 1, **dadurch gekennzeichnet, dass** die Schalenteile (6) und (7) miteinander durch eine gelenkige Verbindung verbunden sind.

3. Handreinigungsinstrument nach Anspruch 2, **dadurch gekennzeichnet, dass** die Schalenteile (6) und (7) mit einem Scharnier (8) gelenkig miteinander verbunden sind.

4. Handreinigungsinstrument nach Anspruch 1 oder 2, **dadurch gekennzeichnet, dass** das Verriegelungsteil (13, 5) eine Klinkenvorrichtung ist.

5. Handreinigungsinstrument nach Anspruch 4, **dadurch gekennzeichnet, dass** die Klinkenvorrichtung ein Ende (13) einer ein Schalenteil (7) verlängernden Achse aufweist, das unter Kraftaufwand in eine Ausnehmung (5) des Endes (4) des Stäbchens (2) einführbar ist, das mit dem anderen Schalenteil (6) verbunden ist.

6. Handreinigungsinstrument nach einem der voranstehenden Ansprüche, **dadurch gekennzeichnet, dass** das Handreinigungsinstrument aus einem einzigen Stück gleichen Materials ausgeführt ist.

7. Handreinigungsinstrument nach einem der voranstehenden Ansprüche, **dadurch gekennzeichnet, dass** das Reinigungsteil (3) in der Anwendungsanordnung zum Verformen bei vorzeitigem Kontakt mit einem Anschlag ausgebildet ist.

8. Handreinigungsinstrument nach einem der voranstehenden Ansprüche, **dadurch gekennzeichnet, dass** die Öffnungen (15) Längsschlitze sind, die die Schalenteile (6) und (7) in einzelne nachgiebige Lamellen (14) unterteilen, die an ihren Enden untereinander verbunden sind.

9. Handreinigungsinstrument nach Anspruch 8, **dadurch gekennzeichnet, dass** die Lamellen (14) abgeflacht sind und Kanten (16) aufweisen, deren Ränder mit Graten (17) ausgebildet sind, mit denen die Wände der zu reinigenden Leitung abschabbar sind.

10. Handreinigungsinstrument nach Anspruch 8 oder 9, **dadurch gekennzeichnet, dass** die Lamellen (14) bei Drehen des Stäbchens (2) um sich selbst eine Verwindungs- oder Bohrwirkung ausüben, die durch schraubenförmige Handhabung den Abtrag von angewachsenen Ablagerungen gestattet.

11. Handreinigungsinstrument nach einem der vorstehenden Ansprüche, **dadurch gekennzeichnet, dass** der freie Innenraum (18) der Schalenteile (6) und (7) ein absorbierendes Element umschließt.

12. Handreinigungsinstrument nach einem der vorstehenden Ansprüche, **dadurch gekennzeichnet, dass** der freie Innenraum (18) der Schalenteile (6) und (7) eine aktive Substanz umschließt, die bei der Anwendung des Handreinigungsinstruments austritt.

13. Handreinigungsinstrument nach Anspruch 12, **dadurch gekennzeichnet, dass** die aktive Substanz von einem absorbierenden Material aufgenommen ist.

14. Handreinigungsinstrument nach Anspruch 12, **dadurch gekennzeichnet, dass** die aktive Substanz in einem Behältnis bevorratet ist und bei der Anwendung des Handreinigungsinstruments austritt.

15. Verfahren zur Herstellung eines Handreinigungsinstruments nach einem der voranstehenden Ansprüche, **dadurch gekennzeichnet, dass** das Stäbchen (2) und das oder die Reinigungsteil(e) (3) an einem Stück und in einem einzigen Spritzgussvorgang ausgebildet werden.

16. Verfahren nach Anspruch 15, **dadurch gekennzeichnet, dass** ein absorbierendes Element, ein getränktes Material oder ein Produktvorrat in wenigstens einen der freien Innenräume (18) der Schalenteile (6) und (7) eingeführt wird.

17. Verfahren nach Anspruch 15 oder 16, **dadurch gekennzeichnet, dass** der freie Innenraum oder die freien Innenräume (18) durch aneinander anliegendes Verbinden der Schalenteile (6) und (7) geschlossen wird beziehungsweise werden und dass die Schalenteile (6) und (7) in der Anwendungsanordnung mittels des Verriegelungsteiles (13, 5) fest miteinander verbunden werden.

## Claims

1. Manual curetting instrument formed by a rod (2) and at least one curetting means (3) integral with one of the ends (4) of the rod (2), **characterised in that** the curetting means (3) is constituted by at least two shell parts (6) and (7) which have openings (15) and can be attached to one another in such a way as to form a complete hollow shell (12) and to delimit a free closed internal space (18) which is capable of being occupied by an absorbent element, an impregnated material or a stock of product, the shell parts (6) and (7) being held in this position of use by a locking means (13, 5).

2. Manual curetting instrument as claimed in Claim 1, **characterised in that** the shell parts (6) and (7) are connected to one another by an articulated joint.

3. Manual curetting instrument as claimed in Claim 2, **characterised in that** the shell parts (6) and (7) are connected to one another and articulated by a hinge (8).

4. Manual curetting instrument as claimed in Claim 1 or 2, **characterised in that** the locking means (13, 5) is a snap fitting device.

5. Manual curetting instrument as claimed in Claim 4, **characterised in that** this snap fitting device is constituted by an axial end (13) extending one of the shell portions (7) which is to be introduced by force into a cut-out (5) at the end (4) of the rod (2) to which the other shell part (6) is joined.

6. Manual curetting instrument as claimed in any one of the preceding claims, **characterised in that** this curetting instrument is made in one piece from the same material.

7. Manual curetting instrument as claimed in any one of the preceding claims, **characterised in that** the curetting means (3), in the configuration for use, is capable of deformation when it is in frontal abutting contact.

8. Manual curetting instrument as claimed in any one of the preceding claims, **characterised in that** the openings (15) are longitudinal slots which divide the shell parts (6) and (7) into individual flexible strips (14) which are connected to one another at each of their ends.

9. Manual curetting instrument as claimed in Claim 8, **characterised in that** the strips (14) are flat and have sides (16) of which the rims are shaped into sharp edges (17) by which they scrape the walls of the duct to be cleaned.

10. Manual curetting instrument as claimed in Claim 8 or 9, **characterised in that** during the rotation of the rod (2) upon itself the strips (14) have a twisting or spinning effect which enables the removed deposits to be extracted by helical entrainment.

11. Manual curetting instrument as claimed in any one of the preceding claims, **characterised in that** the free space (18) inside the shell parts (6) and (7) encloses an absorbent element.

12. Manual curetting instrument as claimed in any one of the preceding claims, **characterised in that** the free space (18) inside the shell parts (6) and (7) encloses an active substance which escapes from the curetting instrument during use thereof.

13. Manual curetting instrument as claimed in Claim 12, **characterised in that** the active substance is impregnated into an absorbent material.

14. Manual curetting instrument as claimed in Claim 12, **characterised in that** the active substance is enclosed in a container and is released in the course of the use of the curetting instrument.

15. Method of manufacturing a manual curetting instrument as claimed in the preceding claims, **characterised in that** the rod (2) and the curetting means (3) are formed in one piece and in one single stage by injection moulding.

16. Method as claimed in Claim 15, **characterised in that** an absorbent element, an impregnated material or a stock of product is introduced into at least one of the free spaces (18) inside the shell parts (6) and (7).

17. Method as claimed in Claim 15 or 16, **characterised in that** the internal free space(s) (18) is/are closed by attaching the shell parts (6) and (7) alongside one another and locking these shell parts (6) and (7) in the configuration for use with the aid of the locking means (13, 5).
